# EUROPEAN PATENT SPECIFICATION

(11) **EP 0 785 750 B1**
(45) Date of publication and mention of the grant of the patent: **30.06.1999**
(21) Application number: 95929950.4
(22) Date of filing: 24.08.1995
(51) Int. Cl.: A61B 5/117, G06K 9/00

(54) **APPARATUS AND METHOD FOR IMAGING SKIN RIDGES**
VORRICHTUNG UND VERFAHREN ZUR ABBILDUNG DER HAUTRIPPEN
DISPOSITIF ET PROCEDE DE MISE EN IMAGE DES SILLONS DE LA PEAU

(30) Priority: 13.10.1994 GB 9420634
(43) Date of publication of application: 30.07.1997
(73) Proprietor: CENTRAL RESEARCH LABORATORIES LIMITED, Hayes, Middlesex, UB3 1HH (GB)
(72) Inventor: WRIGHT, Graham, Central Research Laboratories, Middlesex UB3 1HH (GB)
(74) Representative: Leaman, Keith
(86) International application number: GB9502010
(87) International publication number: WO9611632

(56) References cited:
- EP-A- 0 040 838
- EP-A- 0 304 092
- US-A- 3 533 823
- US-A- 4 336 998
- US-A- 4 363 691
- US-A- 4 404 926

## Description

This invention relates to an apparatus and method for imaging skin ridges, the apparatus comprising a) a light transmissive body having a surface for receiving a relief pattern from skin ridges, and b) a light source for illuminating the surface to form an optical image of any relief pattern in engagement with the surface.

European patent application EP-0-304,092-A1 (NEC Corporation) discloses apparatus for imaging a fingerprint. The finger is pressed against an optical element which is made of an elastic and/or viscous material. This enables a relief pattern with high contrast to be made, even if the surface of the finger to be imaged is dry.

US patent number 3,533,823 (Ling-Temco-Vought Inc) describes a method and apparatus for making a facsimile of a fingerprint. The apparatus utilises a thermochromic material which exhibits hysteresis in changing reflectivity as a result of a change in temperature. The thermochromic material is heated to above body temperature, where it manifests a reduced reflectivity. Certain portions of the material are cooled as a result of contact by ridges on the finger, so that the cooled portions exhibit a reflectivity which differs from other portions.

US patent number 4,363,691 (Energy Conversion Devices Ltd) also describes apparatus for recording skin ridges using a heat responsive recording medium. A finger is pressed against the outer imaging layer of the recording medium during the application of a short pulse of electromagnetic energy or externally generated heat.

Another known apparatus for imaging fingerprints is disclosed in WO 92/11608. In this apparatus a finger urges a relief pattern against one surface of a transparent plastic sheet. Light from a light source is used to illuminate this relief pattern and the surface through the sheet. A resulting light and dark pattern is formed and light from this light and dark pattern is transmitted to other parts of the sheet by means of total internal reflection to form an optical image of the relief pattern.

Such apparatus can, however, suffer from a number of disadvantages. For example, if the skin is cold it is not very compliant, and this can result in a patchy engagement with the surface. To overcome this problem an elastic layer is sometimes interposed between the finger and the surface. Such layers can however deteriorate in use and require replacement. In the absence of such a layer, sweat naturally occurring on the finger tip usually provides good optical coupling between the finger and the surface. Unfortunately, if the finger is very clear and/or cold there may be little surface sweat or other moisture on the finger. Under such conditions the optical coupling between the fingerprint relief pattern and the surface can be poor, resulting in unreliable skin ridge imaging.

It is an object of the present invention to provide an apparatus and method for imaging skin ridges which can enable the above disadvantages to be mitigated.

According to a first aspect of the invention, there is provided an apparatus as defined in the first paragraph above characterized in that the apparatus is provided with light transmissive heating means being constructed such that the skin ridges are heated for at least part of the time when the relief pattern is in engagement with the surface, so as to improve optical coupling between the surface and the relief pattern.

This arrangement can provide the advantage of increasing sweat production at the skin ridges thereby improving optical coupling to the surface. Heating the skin ridges can also increase the flexibility of the skin of the finger, resulting in more complete and/or reproducible relief pattern imaging. A deformable layer may optionally be present between the skin ridges and the surface.

The heating means is preferably provided adjacent the surface. This can allow the light which generates the optical image to pass through the heating means without masking areas of the relief pattern.

The apparatus advantageously comprises a heating element carried by the light transmissive body, such as for example a layer of indium tin oxide through which an electric current is passed to cause the surface to heat up.

As an alternative, or in addition, other heating means may be employed. For example an electromagnetic radiation absorptive layer may be provided adjacent the surface together with an electromagnetic radiation source for illuminating the layer. Alternatively, or in addition, direct heating of the skin ridges by an infra-red or visible light source may be employed.

Preferably the relief pattern is constituted by the pattern of ridges on the surface of the skin of the pad of a digit (i.e. a finger or thumb or toe) or a palm print. Alternatively, a deformable lamina may be provided between the skin ridges and the surface of the light transmissive body. In this case the relief pattern is formed in the deformable layer. This is preferably achieved by the skin ridges being urged into engagement with the deformable layer.

According to a second aspect of the invention, there is provided a method as defined in claim 7.

Embodiments of the invention will now be described, by way of example only, with reference to the accompanying diagrammatic drawings, in which - Figure 1 shows a first embodiment according to the invention, Figure 2 shows a second embodiment according to the invention, and Figure 3 shows a flow diagram of a method according to the invention.

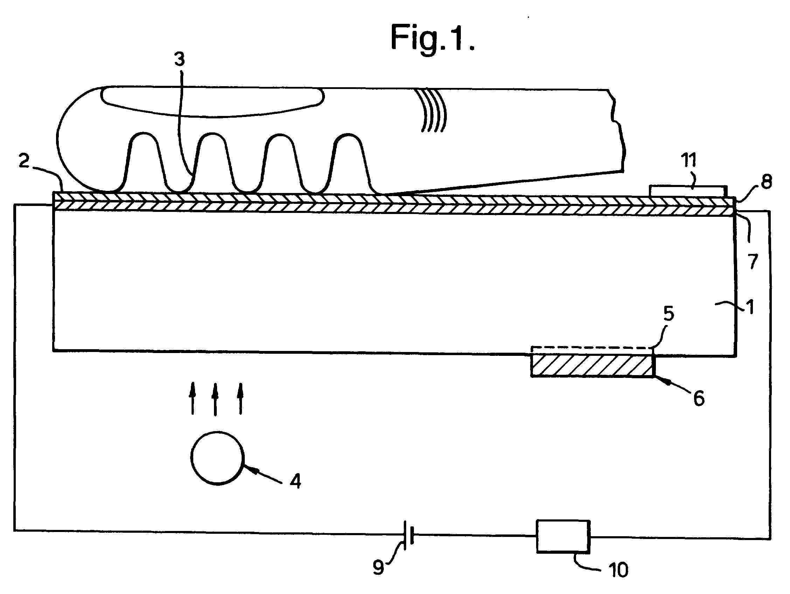

In Figure 1, apparatus for imaging skin ridges comprises a) a light transmissive body (1), in the present case a transparent sheet made from Schott BK7 glass together with surface layers which will be described below. The body has a surface (2) for receiving a relief pattern (3) from skin ridges, in the present case a fingerprint, which in operation is urged against the surface. The apparatus also comprises b) a light source (4) for illuminating the surface to form an optical image (5) of any relief pattern in engagement with the surface. In the present example the optical image is formed on a major surface of the sheet and is directly viewable because it is formed in a thin light-transmissive layer of white paint (6) which scatters light in all directions. The surface (2) is provided with heating means (7, 8) to heat the skin ridges when the relief pattern is in contact with the surface. In the present case the heating means comprises a transparent layer (7) of indium tin oxide (ITO), 100nm thick, deposited on the sheet of glass by r.f. sputtering, together with an overlying transparent layer (8) of r.f. sputtered silicon dioxide (also 100nm thick) which acts as a passivation layer. The ITO layer has a sheet resistance of 10 ohms per square, and is connected to a source of electrical power (9) via control means (10), which controls the delivery of power. The layer (7) thus acts as an electrical heating element which raises the temperature of the surface. The control means (10) co-operates with a temperature sensing element (11) to control the electrical power supplied and hence the temperature of the surface in operation. In the present example the temperature sensing element comprises a thermocouple (11) cemented to the surface (2) in a position just outside the area which receives the relief pattern. The temperature sensing element is not covered by the passivation layer (8) in the present example. In the example of Figure 1, the thermocouple comprises a type N thermocouple, as defined in British Standard BS 4937 part 8 (1986), and obtainable in Britain from RS Components Ltd. This thermocouple is used together with the control means (10) to vary the current in the ITO layer to maintain a desired surface temperature whilst the relief pattern is being imaged. In the present example the surface is heated for the whole time that the relief pattern is in contact with the surface. The control means includes a switch which is dosed when the skin ridges contact the surface and opened when they are removed. This takes the form of a microswitch which is closed by the light transmissive body being urged against it by the finger in use, and opened by the action of a biasing means, such as for example a spring, which biases the switch into its open position in the absence of finger pressure. The surface in the above example is heated in operation to a temperature of 37°C, so that it is not heated above its softening point.

The apparatus described above bears some resemblance to that disclosed in US 4,336,998. In this document the sensing surface is elastic and is provided with a heating element. However, the heating element is only energized after the finger has been removed from the surface and after the surface has been provided with an electrical charge. Thus no heat energy is transferred to the skin ridges by the heating means in operation. When the heater is energized in US 4,336,998, the surface is heated above its softening point.

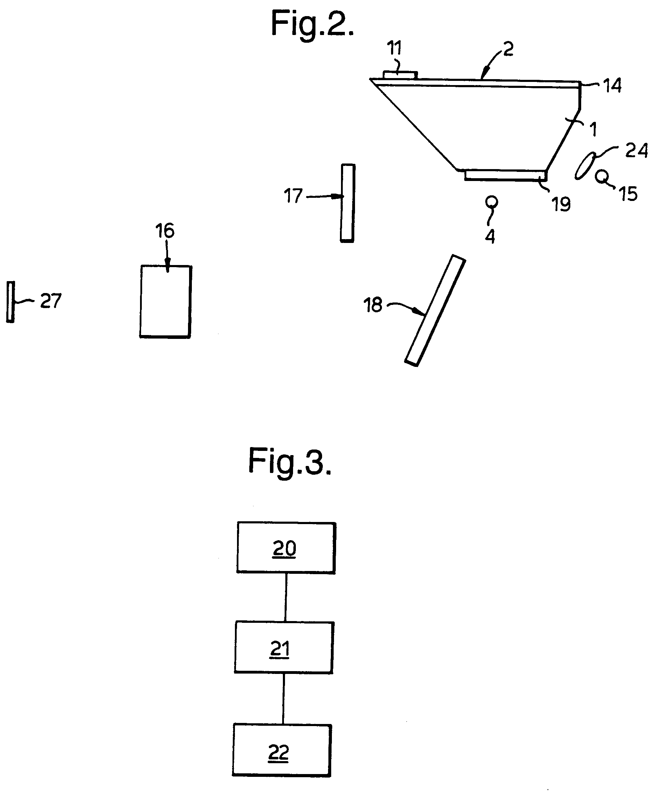

The apparatus of Figure 1 is operated using a method comprising the following steps:
a) urging a relief pattern from skin ridges into engagement with a surface
b) heating the skin ridges, and
c) generating an optical image of the relief pattern.
This method is illustrated in the flow diagram of Figure 3. In this diagram the blocks have the following significance. Block 20 corresponds to step a), block 21 corresponds to step b) and block 22 corresponds to step c).

In step a) a relief pattern from skin ridges (constituted in the present example by a fingerprint) is urged against the surface (for example (2) in Figure 1) so that the relief pattern makes contact with said surface. The relief pattern which makes contact with the surface in the present example is the skin ridges on the pad of a finger, but it may alternatively comprise a deformable lamina (for example latex in the form of a thin sheet) which is deformed by the action of the pad of a digit to give a relief pattern corresponding to the fingerprint when in engagement with the surface (2).

In step b), when the relief pattern is in contact with the surface, the skin ridges are heated. To achieve this the heating means is activated thereby heating up the surface. The heated surface transfers heat by means of thermal conduction to the relief pattern constituted by the skin ridges in the present example. This results in the skin of the finger rising in temperature as it is in contact with the heated surface (or, alternatively in contact with a deformable layer which is itself in contact with the heated surface if a deformable layer is present). The temperature to which the surface is heated is sufficiently high that the skin of the pad of the digit becomes more flexible and/or the skin pores secret sweat. The temperature range is preferably in the range 25°C to 50°C, very preferably in the range 30°C to 39°C. The temperature must not be so high that finger contact produces discomfort within a few seconds.

In step c) (22), light illuminates the surface whilst the skin ridges are in contact with the surface. The light is totally internally reflected in the body to form an image of the relief pattern, in the present example a directly viewable real optical image on a major surface of the light transmissive sheet.

A second example of apparatus according to the first aspect of the invention is shown in Figure 2. In this Figure there are a number of elements which correspond with elements shown and numbered in Figure 1. These elements are consequently labelled with the same numerals in Figure 2 as they have in Figure 1. In this example, the light-transmissive body (1) is in the form of a transparent prism made of Schott BK7 glass, having a thin electromagnetic radiation absorptive layer (14) 1mm thick constituted by a Schott neutral density filter (in the present example made from glass) adjacent the surface. This layer is fixed to the glass substrate using an optical coupling compound such as Canada balsam. Electromagnetic radiation from a source (15) is focussed by a lens (24) onto the filter which absorbs energy, thereby increasing the temperature of the layer and hence the surface (2). The source in this embodiment comprises a filament lamp or halogen lamp, although other light sources or sources of infra-red radiation may be used as an alternative.

Once again a thermocouple (11) may be used in conjunction with a control means (not shown) to maintain the temperature of the surface (2) at a desired value whilst a relief pattern corresponding to a fingerprint (not shown) is imaged. This temperature control is achieved in the present case for example by reducing the intensity of the electromagnetic radiation emitted by the source (15). Alternatively, or in addition the source may be flashed on and off to reduce the average intensity in a given time interval. The relief pattern is imaged by a CCD (27) together with a lens (16), having a focal length of 35 mm, arranged to receive light from the light source (4) which has illuminated the surface (2) and the relief pattern (3) through the light transmissive body (1) and a diffuser (19). The light from the body is arranged to be reflected from two mirrors (17 and 18) on its way to the lens (16) and CCD. The preferred temperature of the surface of the embodiment of Figure 2 in operation is 33°C. In this second example, the illumination of the layer by the electromagnetic radiation source (15) is stopped for part of the time when the skin ridges are in contact with the surface to allow the light source (4) to generate an optical image of the relief pattern with no interference from the electromagnetic radiation source. As an alternative the two sources may be constituted by a single source of light.

In the embodiment of Figure 2 the image of the skin ridges is not directly viewable, as it requires an additional optical system (16 and 17) comprising a lens and/or screen, unlike the apparatus shown in Figure 1.

In the apparatus described in the examples above, the surface is made from a material which does not soften at the temperatures to which the surface is heated (i.e. 25°C - 50°C or 30°C - 39°C). As an alternative, materials which will soften in the above temperature ranges may be employed if desired. As a further alternative a material having resilience may be employed. Although in the above examples, a temperature sensing device and temperature control circuit has been employed, it is possible to use the apparatus without such means provided the heating means is carefully designed to provide just enough heat energy to balance heat losses at the desired operating temperature.

Although in the above cases the surface (2) is flat, a curved surface may be used as an alternative.

In summary, an apparatus for imaging skin ridges comprises a light transmissive body having a surface for receiving a relief pattern from, for example, the pad of a finger, and a light source which illuminates the surface and generates an optical image of the relief pattern. A light transmissive heating element is provided adjacent the surface of the body to heat the skin ridges in operation. The increase in the temperature of the skin makes it more flexible. It also promotes sweat secretion. Both factors improve the optical coupling between the finger and the surface resulting in more reliable fingerprint imaging.

## Claims

1. Apparatus for imaging skin ridges, comprising a) a light transmissive body (1) having a surface (2) for receiving a relief pattern (3) from skin ridges, and b) a light source (4) for illuminating the surface to form an optical image (5) of any relief pattern in engagement with the surface, characterized in that the apparatus is provided with light transmissive heating means (7,8) being constructed such that the skin ridges are heated for at least part of the time when the relief pattern is in engagement with the surface, so as to improve optical coupling between the surface (2) and the relief pattern (3).

2. Apparatus as claimed in Claim 1 in which the heating means (7,8) is provided adjacent the surface (2).

3. Apparatus as claimed in Claim 1 or Claim 2 in which the heating means (7,8) comprises a heating element (7) carried by the body (1).

4. Apparatus as claimed in Claim 1 in which the heating means (7,8) comprises an electromagnetic radiation absorptive layer (14) adjacent the surface (2) and an electromagnetic radiation source (15) for illuminating the layer through the body (1).

5. Apparatus as claimed in any preceding claim further comprising control means (10) being constructed to control the heating means (7,8) such that in operation it supplies heat energy for at least a part of the time when a relief pattern (3) is in engagement with the surface (2).

6. Apparatus as claimed in any preceding claim in which the relief pattern (3) is formed by the skin ridges in a deformable lamina.

7. A method of imaging skin ridges, comprising the steps of:
a) urging a relief pattern (3) from skin ridges into engagement with a surface (2)
b) heating the surface (2) using a light transmissive heating means (7,8) thereby heating the skin ridges for at least part of the time when the relief pattern (3) is in engagement with the surface, thereby improving optical coupling between the surface (2) and the relief pattern (3),
characterised by
c) generating an optical image of the relief pattern by illuminating said skin ridges while in engagement with said surface.

## Patentansprüche

1. Vorrichtung zur Abbildung der Hautrippen enthaltend a) einen lichtdurchlässigen Körper (1) mit einer Oberfläche (2) zur Aufnahme eines Reliefmusters (3) von Hautrippen, und b) eine Lichtquelle (4) zur Beleuchtung der Oberfläche zum Erlangen einer optischen Abbildung (5) eines beliebigen Reliefmusters, das sich mit der Oberfläche in Kontakt befindet, dadurch gekennzeichnet, daß die Vorrichtung mit lichtdurchlässigen Heizmitteln (7, 8) versehen ist, die so ausgebildet sind, daß die Hautrippen zumindest über einen Teil der Zeit, während sich das Reliefmuster mit der Oberfläche in Kontakt befindet, aufgeheizt werden, um die optische Kopplung zwischen der Oberfläche (2) und dem Reliefmuster (3) zu verbessern.

2. Vorrichtung nach Anspruch 1, wobei die Heizmittel (7, 8) an die Oberfläche (2) angrenzend vorgesehen sind.

3. Vorrichtung nach Anspruch 1 oder Anspruch 2, wobei die Heizmittel (7, 8) ein von dem Körper (1) getragenes Heizelement (7) enthalten.

4. Vorrichtung nach Anspruch 1, wobei die Heizmittel (7, 8) eine elektromagnetische Strahlung absorbierende Schicht (14) angrenzend an die Oberfläche (2) sowie eine elektromagnetische Strahlungsquelle (15) zur Beleuchtung der Schicht durch den Körper (1) enthalten.

5. Vorrichtung nach einem der vorhergehenden Ansprüche enthaltend ferner Steuermittel (10) ausgebildet zur Steuerung der Heizmittel (7, 8) derart, daß diese im Betrieb über zumindest einen Teil der Zeit, während der sich ein Reliefmuster (3) mit der Oberfläche (2) in Kontakt befindet, Heizenergie liefern.

6. Vorrichtung nach einem der vorhergehenden Ansprüche, wobei das Reliefmuster (3) durch die Hautrippen in einem verformbaren Plättchen gebildet wird.

7. Verfahren zur Abbildung der Hautrippen umfassend die Schritte:
a) In Kontakt Bringen eines Reliefmusters (3) von Hautrippen mit einer Oberfläche (2);
b) Beheizen der Oberfläche (2) unter Verwendung lichtdurchlässiger Heizmittel (7, 8) und damit Beheizen der Hautrippen über wenigstens einen Teil der Zeit, während der sich das Reliefmuster (3) mit der Oberfläche in Kontakt befindet; hierdurch Verbesserv der optischen Kopplung zwischen der Oberfläche (2) und dem Reliefmuster (3), gekennzeichnet durch
c) Erzeugung einer optischen Abbildung des Reliefmusters durch Beleuchtung der Hautrippen, während des Kontakts mit der Oberfläche.

## Revendications

1. Appareil de mise en image des sillons de la peau, comprenant :
a) un corps (1) laissant passer la lumière, ayant une surface (2) pour recevoir une forme en relief (3) de sillons de la peau, et
b) une source lumineuse (4) pour éclairer la surface pour former une image optique (5) de n'importe quelle forme en relief en contact ave la surface,
caractérisé en ce que l'appareil est doté de moyens chauffants (7, 8) laissant passer la lumière, conçus de façon telle que les sillons de la peau sont chauffés pendant au moins le moment où la forme en relief est en contact avec la surface, de façon à améliorer le couplage optique entre la surface (2) et la forme en relief (3).

2. Appareil selon la revendication 1, dans lequel les moyens chauffants (7, 8) sont prévus en étant adjacents à la surface (2).

3. Appareil selon la revendication 1 ou 2, dans lequel les moyens chauffants (7, 8) comprennent un élément chauffant (7) supporté par le corps (1).

4. Appareil selon la revendication 1, dans lequel les moyens chauffants (7, 8) comprennent une couche (14) absorbant le rayonnement électromagnétique, adjacente à la surface (2), et une source (15) de rayonnement électromagnétique pour éclairer la couche à travers le corps (1).

5. Appareil selon l'une quelconque des revendications précédentes, comprenant en outre un moyen de commande (10) conçu pour commander les moyens chauffants (7, 8) de façon telle, qu'en cours de fonctionnement, ils fournissent de l'énergie thermique pendant au moins le moment où une forme en relief (3) est en contact avec la surface (2).

6. Appareil selon l'une quelconque des revendications précédentes, dans lequel la forme en relief (3) est formée par les sillons de la peau dans une lamelle déformable.

7. Procédé de mise en image des sillons de la peau, comprenant les étapes consistant :
a) à appliquer une forme en relief (3) de sillons de la peau, en contact avec une surface (2),
b) à chauffer la surface (2) en utilisant des moyens chauffants (7, 8) laissant passer la lumière, chauffant ainsi les sillons de la peau pendant au moins le moment où la forme en relief (3) est en contact avec la surface, améliorant ainsi le couplage optique entre la surface (2) et la forme en relief (3),
caractérisé
c) par la génération d'une image optique de la forme en relief en éclairant lesdits sillons de la peau, alors que la forme en relief est en contact avec ladite surface.
